# EUROPEAN PATENT APPLICATION

(11) **EP 2 436 757 A2**
(43) Date of publication of application: **04.04.2012**
(21) Application number: 10780788.5
(22) Date of filing: 26.05.2010
(51) Int. Cl.: C12N 5/04, C12N 5/02

(54) **PLANT STEM CELL DERIVED FROM CAMBIUM OF FAMILY ASTERACEAE AND METHOD FOR THE ISOLATED CULTURING THEREOF**

(30) Priority: 26.05.2009 KR 20090045953
(71) Applicant: Unhwa Corporation, Jeollabuk-do 561-211 (KR)
(72) Inventor: KIM, Il Suk, Jeonju-si Jeollabuk-do 561-222 (KR); PAEK, Jin Soo, Jeonju-si Jeollabuk-do 561-792 (KR); JIN, Young Woo, Jeonju-si Jellabuk-do 561-808 (KR); LEE, Eun Kyong, Iksan-si Jeollabuk-do 570-976 (KR); HONG, Sun Mi, Jeonju-si Jeollabuk-do 561-231 (KR)
(74) Representative: Kiddle, Simon John
(86) International application number: PCT/KR2010/003340
(87) International publication number: WO 2010/137877

(57) **Abstract**

The present invention relates to a stem cell derived from a cambium of the family *Asteraceae* and a method of isolating and culturing the same. The inventive stem cell derived from the cambium of the family *Asteraceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell and a culture thereof have not only the effect of inhibiting the effect of inhibiting NO production caused by treatment with LPS (lipopolysaccharide), but also the effect of inhibiting the expression COX-2 gene (proinflammatory cytokine), and are thus useful as an effective anti-inflammatory composition.

## Description

### TECHNICAL FIELD

The present invention relates to a stem cell derived from a cambium of family *Asteraceae* and a method of isolating and culturing the same.

### BACKGROUND ART

Plants were recognized as food resources in the past, but the meaning thereof is currently expanded to include a source for a wide range of chemical substances, including drugs, perfumes, pigments, agricultural chemicals, dyes and etc. Particularly, because most useful substances derived from plants have physiological activities, including antiviral, antibacterial, anticancer and antioxidant activities, plants are considered as ideal resources which can be developed into novel drugs, and active studies are in progress to elucidate the relationship between the chemical structures and activities of many plant-derived substances.

However, physiologically active substances are difficult to develop into drugs, and the main reasons therefore are as follows. First, the contents of physiologically active substances in plants are very limited. Second, the growth rate of plants is very slow. Third, physiologically active substances derived from plants are present only in specific organs of plants in small amounts. Fourth, environmental problems associated with the destruction of nature are involved. Fifth, physiologically active substances derived from plants have very complicated chemical structures, such that multi-step polymerization processes are required, thus causing economic problems of high production costs. For these reasons, it was very difficult to stably supply physiologically active substances derived from plants for commercialization.

Meanwhile, a plant cell culture method, one of bioengineering techniques, has been evaluated for a long time as the most ideal technique which can supply plant-derived useful substances without causing environmental problems. According to Korean Patent Publication No. 1995-0000870 (January 3, 1995), the production of useful substances by plant cell culture technique provides many advantages over methods of extracting useful substances directly from plants. Particularly, the plant cell culture method has been regarded as an optimal method which allows continuous production without being influenced by external environments so as to solve outstanding problems such as ecosystem destruction, unlike the prior extraction methods. However, despite great interest and effort in plant cell culture, examples which succeeded in industrializing the plant cell culture are still insufficient. This is because the variations in cell growth and productivity in a number of plant cell cultures still remain as a major problem.

In a case of that plant cells are used in plant expression systems, a de-differentiation process is necessarily carried out in advance in order to convert tissues into a cell line having the ability to divide because the tissues which differentiated from the plant cells, for example, leaves, stems and seeds, are permanent tissues whose cells no longer divide. The de-differentiation process means de-differentiating tissues or cells, which have already been differentiated so as to perform specific functions, when a plant tissue or organ is cultured. However, during this de-differentiation process, serious changes in the cell line can occur due to chromosomal variations.

Particularly, the production of useful substances through plant cell culture can be industrialized, only when rapid cell growth and high metabolite productivity are stably maintained during a long-term culture period. However, most cells undergo many variations different from the original due to subculture. Thus, it is urgent to overcome this problem of variations in cells and to develop a method for acquiring a genetically stable cell line in producing useful substances through plant cell culture.

Korean Patent Registration 10-0533120 developed by some of the present inventors discloses a method of inducing callus using the cambium collected from the stem of a plant. However, in this registered patent, the callus is merely induced from the cambium of woody plant stem. This registered patent still has the problem of variation caused by dedifferentiation because the callus is a tissue formed through a dedifferentiation process.

Furthermore, some of the present inventors developed the invention of PCT/KR 2006/001544, which solves the problem of variation caused by dedifferentiation and relates to a method for providing cell lines that can stably proliferate and have high genetic stability. Meanwhile, Artemisia, a member of the genus *Artemisia* of the family *Asteraceae,* is widely known as a medicinal plant having blood purifying, detoxifying and immune enhancing effects. In this case of Artemisia, there has been a need to obtain a cell line that overcomes the problem of variation caused by dedifferentiation, can stably proliferate and have high genetic stability.

Accordingly, the present inventors have made extensive efforts to obtain a useful plant cell line by isolating a stem cell derived from a cambium of family *Asteraceae.* As a result, the present inventors have isolated a stem cell derived from the cambium of the family *Asteraceae* and have found that the isolated stem cells stably proliferate and do not vary during culture, thereby completing the present invention.

### DISCLOSURE OF INVENTION

Therefore, it is an object of the present invention to provide a stem cell derived from a cambium of family *Asteraceae* without undergoing a dedifferentiation process, and a method for isolating the stem cell.

To achieve the above object, the present invention provides a stem cell derived from a cambium of family *Asteraceae,* in which the stem cell is an innately undifferentiated cell without going through dedifferentiation.

The present invention also provides a method of isolating a stem cell derived from a cambium of family *Asteraceae,* the method comprising the steps of:
(a) obtaining a tissue comprising a cambium from a plant of the family *Asteraceae;*
(b) culturing the obtained cambium-comprising tissue in a medium; and
(c) isolating a cell from the cambium, thereby obtaining a stem cell derived from the cambium.

The present invention also provides an anti-inflammatory composition containing any one or more of the stem cell derived from the cambium of the family *Asteraceae,* an extract thereof, a lysate thereof, and a culture thereof.

The present invention also provides a functional food for preventing or alleviating inflammation, which contains any one or more of the stem cell derived from the cambium of the family *Asteraceae,* an extract thereof, a lysate thereof, and a culture thereof.

Other features and embodiments of the present invention will be more apparent from the following detailed descriptions and the appended claims

### BRIEF DESCRIPTION OF THE DRAWINGS

FIG. 1A is a photograph showing an Artemisia material, FIG. 1B is a photograph showing an explant comprising cambium tissue, and FIG. 1C is a photograph showing cells induced from the cambium.
FIG. 2 is a photograph showing a solid culture of artemisia cambium-derived stem cells according to the present invention.
FIG. 3A is a photograph of a chrysanthemum material, and FIG. 3B is a photograph showing the results of culturing an explant comprising the cambium tissue of a chrysanthemum.
FIG. 4A is a micrograph showing the degree of aggregation of stem cells during culture, and FIG. 4B is a micrograph showing the degree of aggregation of cells in a callus derived from the hypocotyl tissue of artemisia. Scale bar in FIG. 4A: 75 µm, and scale bar in FIG. 4B: 250 µm.
FIG. 5A is a micrograph showing a stem cell according to the present invention, observed after staining of the vacuole with neutral red, FIG. 5B is a micrograph showing a callus derived from the hypocotyl tissue of artemisia. Scale bar at the bottom of the right side of each photograph: 20 µm.
FIG. 6A is a photograph showing mitochondria in a stem cell according to the present invention, and FIG. 6B is a photograph showing mitochondria in a callus derived from the hypocotyl tissue of Artemisia.
FIG. 7 shows a growth curve of stem cells according to the present invention.
FIG. 8 is a set of photographs showing the result of culture for 12 days in a 3-liter air-lift bioreactor (FIG. 8A) and a 20-liter air-lift bioreactor (FIG. 8B).
FIG. 9 is a photograph showing a tracheary element (TE) that differentiated from a stem cell according to the present invention.
FIG. 10 is a graph of the degree of inhibition of NO generation at different concentration of an extract of stem cell cultures according to the present invention.
FIG. 11 is a set of photographs of gels showing the degree of inhibition of COX-2 gene expression at different concentrations of stem cells according to the present invention and an extract of a culture thereof (A: growth phase 0.5 g/ml; B: elicitation phase 0.5 g/ml; C: growth phase 2 mg/ml; D: elicitation phase 2 mg/ml).

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein is well known and commonly employed in the art.

The definition of main terms used in the detailed description of the invention is as follows.

As used herein, the vascular "cambium" refers to a lateral meristem located within vascular tissue of a plant and is located on a stem and a root. The thickening growth of plants occurs by the activity of the cambium, and as a result, giant plants having more than 11,000 years of the growth rings may exist. Embryologically, the vascular cambium originates from the procambium, and thus is the same meristem which has been gradually differentiated with meristematic continuity (Jae-Doo LEE and seven others, "Plant Morphology", Academy Book Publishing Co, Chapter 10, 1993). In the present invention, the term "cambium" is meant to include a procambium. Such cambium and procambium are primary meristem, and thus in the present invention, it is obvious that the use of such cambium and procambium provides the same effect.

As used herein, the term "stem cell" refers to an innately undifferentiated cell without passing through a dedifferentiation process and is thus genetically highly stable.

As used herein, the term "lysate" refers to a cell lysate obtained by disrupting cells through a chemical method, for example, using a detergent, or a physical method. The term "extract" of a cell line refers to a substance obtained by dissolving cells in a solvent and being isolated, and the extract can be concentrated through distillation or evaporation. Also, the term "culture medium" of a cell line refers to a cell medium solution from which cells have been removed after culturing the cells. Additionally, the term "culture" as used herein refers to a substance including a culture medium and/or a cultured cell line, wherein the cultured cell line is meant to include all cell lines which differentiate under culture conditions or which have improved ability to produce and/or secrete useful substances

As used herein, the term "innately undifferentiated" means that cells are not present in an undifferentiated state through a dedifferentiation process, but are originally maintained in a pre-differentiated state.

As used herein, the term "callus" refers to cells or a cell colony which has not differentiated through a dedifferentiation process (PNAS, 99(25):15843, 2002).

In one aspect, the present invention is directed to a stem cell derived from a cambium of family *Asteraceae,* in which the stem cell is an innately undifferentiated cell without going through dedifferentiation.

When leaf, stem or root portions which are already differentiated tissues are used, they undergo a dedifferentiation process in which they are rejuvenated to their undifferentiated state in order to form a callus. During the dedifferentiation process, a mutation in somatic cells appears to cause cell unstability. Accordingly, the present inventors have conducted studies on a plant cell system showing little or no somaclonal variation, and as a result, have found that, when a cell line is derived specifically only from the meristem cambium, the ability of the metistem itself vigorously to divide can be used, and thus a cell line, which has no somaclonal variation and is genetically highly stable and physiologically uniform, can be derived without undergoing a dedifferentiation process. Based on this finding, a cambium-derived stem cell line was isolated.

A stem cell derived from the cambium of the family *Asteraceae* may have at least one of the following characteristics: (a) including a greater number of single cells or including smaller-sized cell aggregates in suspension culture than a dedifferentiated callus of the family *Asteraceae;* (b) having a large number of vacuoles morphologically; (c) having a greater number of mitochondria than the dedifferentiated callus of the family *Asteraceae;* (d) being capable of growing faster and longer than the dedifferentiated callus of the family *Asteraceae;* (e) having lower sensitivity to shear stress in a bioreactor than the dedifferentiated callus of the family *Asteraceae;* and (f) having the capability to differentiate into tracheary elements.

As used herein, the phrase "having a large number of vacuoles" means having a number of vacuoles which is at least two times greater than the number of vacuoles in the dedifferentiated callus of the family *Asteraceae.* In addition, the stem cells according to the present invention have smaller vacuoles than the dedifferentiated callus of the family *Asteraceae.* Furthermore, the phrase "having a greater number of mitochondria" as used herein means having a number of mitochondria which is at least two times greater than the number of mitochondria in the dedifferentiated callus of the family *Asteraceae.* Herein, the stem cells according to the present invention are characterized by having mitochondria having increased activity compared to that of mitochondria in the dedifferentiated callus of the family *Asteraceae,* in which the phrase "having mitochondria having increased activity" means having mitochondria moving actively under a microscope. In addition, the present inventors have found that stem cells derived from the cambium of the family *Asteraceae* have the capacity to differentiate into tracheary elements. The cells derived from the cambium of the family *Asteraceae* according to the present invention have self-renewal ability and pluripotency which are the characteristics of plant stem cells, indicating that the cells according to the present invention are stem cells.

In the present invention, the stem cells may have at least two of the above characteristics (a) to (f), preferably at least three of the above characteristics (a) to (f), and more preferably at least four of the above characteristics (a) to (f). In addition, the stem cells according to the present invention may also have at least five of the above characteristics (a) to (f), or all of the above characteristics (a) to (f).

In another aspect, the present invention provides a method for preparing the stem cells according to the present invention, the method comprising the steps of: (a) obtaining a tissue comprising a cambium from a plant of the family *Asteraceae;* (b) culturing the obtained cambium-comprising tissue in a medium; and (c) isolating a cell from the cambium, thereby obtaining a stem cell derived from the cambium. Step (b) of the method according to the present invention may be performed by culturing the cambium-comprising tissue to induce a cultured cambium proliferated from the cambium, and step (c) of the method may be performed by isolating the cultured cambium to obtain a cambium-derived stem cell. Step (b) of the method may be performed by culturing the cambium-comprising tissue in a medium containing auxin, in which auxin in the medium may be NAA (α-naphtalene acetic acid) or IAA (indole-3-acetic acid). Such auxin may be contained at a concentration of 1-5 mg/ℓ in the medium. Also, step (c) of the method may be performed by isolating the cultured cambium from an amorphous callus layer that proliferated from tissues other than the cambium, thereby obtaining the cambium-derived stem cell.

In one Example of the present invention, artemisia cambium-derived stem cells were isolated from artemisia, a member of the genus *Artemisia* of the family *Asteraceae,* and chrysanthemum cambium-derived stem cells were also isolated from a chrysanthemum, a member of the genus *Chrysanthemum* of the family *Asteraceae,* in the same manner as the isolation of the artemisia cambium-derived stem cells, and were found to have characteristics similar to the artemisia cambium-derived stem cells. Thus, the stem cells according to the present invention are preferably stem cells derived from the cambium of a plant of the genus *Artemisia* or the genus *Chrysanthemum,* and more preferably stem cells derived from the cambium of Artemisia or a chrysanthemum.

Also, in the present invention, the stem cells derived from the cambium of Artemisia, a member of the genus *Artemisia* of the family *Asteraceae,* were found to have anti-inflammatory effects.

In another aspect, the present invention is directed to an anti-inflammatory composition containing any one or more of said stem cell line derived from the cambium of the family *Asteraceae,* an extract thereof, and a lysate thereof.

In the present invention, the culture of the stem cell line is preferably obtained by additionally culturing the stem cell in a medium, which, as elicitors, contains 3-5 wt% of raw sugar or sugar, and/or any one or more of methyl jasmonate, chitosan, phenylalanin, benzoic acid, ABA, salicylic acid and sodium acetate. Herein, the medium preferably contains 3-5 wt% of raw sugar or sugar, and at least one substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

Also, in the present invention, it is possible to use a culture obtained by treating the cell line with elicitors, including UV radiation, heat, ethylene, an antifungal agent, an antibiotic, heavy metal salt and high-concentration salt, and applying physical and chemical stresses thereto.

In the present invention, the extract is preferably obtained using a solvent selected from the group consisting of distilled water, alcohol such as ethanol, acetone, DMSO (dimethyl sulfoxide), and mixed solvents thereof.

In one Examples of the present invention, it was found that the Artemisia cambium-derived stem cell and an extract of a culture thereof have not only the effect of inhibiting NO production caused by treatment with LPS (lipopolysaccharide), but also the effect of inhibiting the expression of COX-2 gene (proinflammatory cytokine), indicating that they have anti-inflammatory effects. Thus, the stem cell derived from the cambium according to the present invention and an extract of a culture thereof will be useful as an effective anti-inflammatory composition.

Thus, even though in the present invention, there is no specific example showing that a composition containing the stem cells or a lysate thereof shows anti-inflammatory effects, it will be obvious to those skilled in the art that the composition containing the stem cells according to the present invention or a lysate thereof can also show anti-inflammatory activity to inhibit inflammation, because the stem cells or an extract of a culture of the stem cells were found to have anti-inflammatory activity as described above.

The anti-inflammatory composition which contains, as an active ingredient, any one or more of the stem cell according to the present invention, an extract thereof, a lysate thereof and a culture thereof, may be provided as a pharmaceutical composition additionally containing at least one pharmaceutically acceptable carrier, excipient or diluent. The stem cell line or the like may be contained in a pharmaceutical composition in a pharmaceutically effective amount depending on disease and its severity, the patient's age, weight, health condition and sex, the route of administration and the period of treatment.

As used herein, the term "pharmaceutically acceptable composition" refers to a composition that is physiologically acceptable and does not cause gastric disorder, allergic reactions such as gastrointestinal disorder or vertigo, or similar reactions, when administered to humans. Examples of said carrier, excipient or diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, magnesium stearate and mineral oils.

The pharmaceutical composition of the present invention may additionally contain fillers, anti-aggregating agents, lubricants, wetting agents, perfumes, emulsifiers and preservatives. Also, the pharmaceutical composition of the present invention may be formulated using a method well known in the art, such that it can provide the rapid, sustained or delayed release of the active ingredient after administration to mammals. The pharmaceutical composition of the present invention may be in the form of powders, granules, tablets, emulsions, syrups, aerosols, soft or hard gelatin capsules, sterile injection solutions, sterile powders, etc.

In still another aspect, the present invention is directed to an a functional food for preventing or alleviating inflammation, which contains, as active ingredients, any one or more of said stem cell derived from the cambium of the family *Asteraceae,* an extract thereof, a lysate thereof, and a culture thereof.

As used herein, the term "functional food" refers to a food, the functionality of which has been improved by adding thereto any one or more of the stem cell according to the present invention, a lysate thereof, an extract thereof, or a culture thereof.

### EXAMPLES

Hereinafter, the present invention will be described in further detail with reference to examples. It will be obvious to a person having ordinary skill in the art that these examples are illustrative purposes only and are not to be construed to limit the scope of the present invention.

In the following Examples, stem cells derived from the cambiums of particularly Artemisia and a chrysanthemum among plants of the family *Asteraceae* were isolated. However, it will be obvious to those skilled in the art that plant stem cells having characteristic similar to those of the stem cells isolated in the Examples can be isolated from other plants of the family *Asteraceae.*

### Example 1: Preparation (1) of stem cells derived from cambium of plant of family Asteraceae - Artemisia

### 1-1: Preparation of plant material

In order to obtain Artemisia cambium-derived stem cells from *Artemisia annua L.,* a member of the family *Asteraceae,* Artemisia stems were collected (the Medicinal Plants Research Institute of Jinan, Korea), and then immediately soaked in 100 mg/L of the antioxidant L-ascorbic acid (DUCHEFA, The Netherlands). Then, they were transported and stored. Meanwhile, in order to obtain the same stem cells from the procambium of *Artemisia annua L.,* twigs in place of stems were collected.

Then, the plant was pretreated with a mixed solution of 0.1% benomyl (Dongbu Hannong Chemical, Korea), 0.1% daconil (Dongbu Hannong Chemical, Korea), 0.1% streptomycin sulphate (DUCHEFA, The Netherlands) and 0.01% cefotaxime sodium (DUCHEFA, The Netherlands) for 1 hour, and then washed with tap water for 30 minutes to remove phenolic compounds and the remaining chemicals. Next, the plant was surface-sterilized in 70% ethanol (DC Chemical, Korea) for 30 sec, 3% hydrogen peroxide (LG Chemical, Korea) for 5 min, 0.5% CLOROX solution for 3 min and 0.1% CLOROX solution for 5 min, and then washed 3-4 times with water.

### 1-2: Preparation of cambium-containing explant from stem and separation of tissue

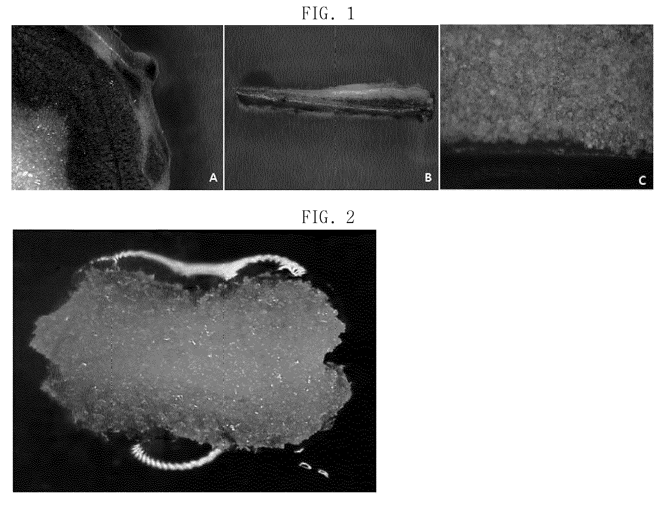

The stem (twig when using the procambium), sterilized in Example 1-1, was cut to a length of 1 cm or less and made cuts to the xylem with a width of 2-5 mm. Then, the xylem was held with tweezers, and tissues consisted of cambium having an excellent ability to divide, phloem and epidermis were peeled off. FIG. 1A is a photograph showing the artemisia material, and FIG. 1B shows a cambium-containing explant obtained from the artemisia material.

### 1-3: Induction of Artemisia cambium-derived stem cells

The cambium-containing explant prepared in Example 1-2 above was laid and cultured in the stem cell induction medium (medium 1) shown in Table 1 below.

**[Table 1]**

| Cell line induction medium (medium 1) | | |
|---|---|---|
| | Composition | Contents(mg/L) |
| | KNO₃ | 2500 |
| | (NO₄)₂SO₄ | 134 |
| | MgSO₄·7H₂O | 121.56 |
| | MnSO₄·4H₂O | 10 |
| | ZnSO₄·7H₂O | 2 |
| | CuSO₄·5H₂O | 0.025 |
| Inorganic salts | CaCl₂·2H₂O | 113.23 |
| | KI | 0.75 |
| | CoCl₂·6H₂O | 0.025 |
| | NaH₂PO₄·H₂O | 130.44 |
| | H₃BO₃ | 3 |
| | Na₂MoO₄·2H₂O | 0.25 |
| | FeNaEDTA | 36.7 |
| | Myo-inositol | 200 |
| | Thiamine-HCl | 20 |
| Vitamin | Nicotinic acid | 2 |
| | Pyridoxine-HCl | 2 |
| | L-ascorbic acid | 50 |
| | Citric acid | 75 |
| Amino acid | L-aspartic acid | 133 |
| | L-arginine | 175 |
| | Glycine | 75 |
| | Proline | 115 |
| Hormone | α-Naphtalene acetic acid | 2 |
| Sucrose | | 10,000 |
| Activated charcoal | | 100 |
| Gelrite | | 2,000 |

The plant growth regulator auxin such as NAA or IAA may be added to the medium at a concentration of 1-5 mg/L, and preferably 2 mg/L. The culture was carried out in a dark room controlled at 25±1 °C.

As a result of culturing the cells as described above, at 4-7 days of initial culture, the division of cells from the cambium was visually observed (FIG. 1C), and after 15 days of culture, an amorphous callus formed by dedifferentiation started to be induced from the layer composed of phloem, cortex and epidermis. After 30 days of culture, the tissue started to be separated into the cultured cambium layer and a layer containing phloem, cortex and epidermis, that is, an amorphous callus layer. After the tissue has been naturally completely separated into the two layers, the layers were separately cultured in different Petri dishes. Specifically, at 4-7 days of initial culture, the division of cells from the cambium was visually observed, and after 15 days of culture, the cambium uniformly divided into a plate-shape could be observed; however, cell division in the phloem did not occur throughout the culture period, and thus only the cells induced from the cambium could be separated from the cells of other tissues.

As can be seen in FIG. 2, the Artemisia cambium-derived stem cells were stably maintained without variations in the growth rate, growth pattern and degree of aggregation of the cells during long-term culture.

### Example 2: Preparation (2) of stem cells derived from cambium of plant of family Asteraceae - chrysanthemum

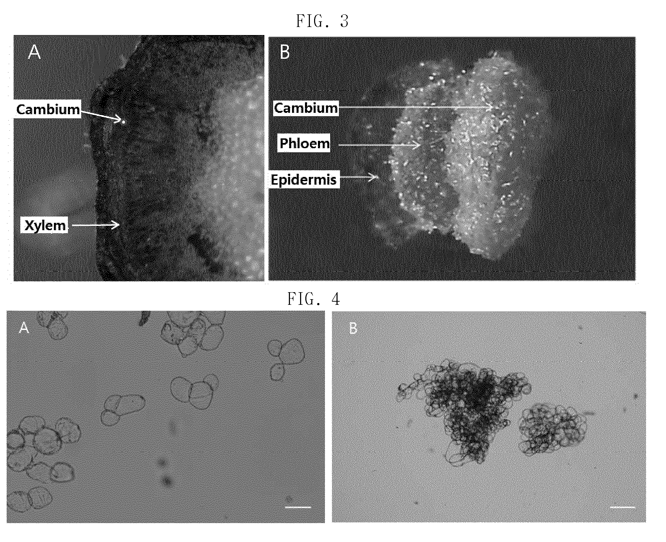

A stem from a chrysanthemum (Dongsan Flower Farming Association, Korea), a member of the genus *Chrysanthemum* of the family *Asteraceae,* was collected, and then treated in the same manner as Example 1-1 above, thus preparing a material (FIG. 3A). Then, a chrysanthemum cambium-containing explant was prepared from the material in the same manner as Example 1-2 above. Then, the explant was planted and cultured in the stem cell induction medium shown in Table 1.

At 4-7 days of initial culture, the division of cells from the cambium was visually observed, and after 10 days of culture, an amorphous callus formed by dedifferentiation started to be induced from the layer composed of phloem, cortex and epidermis. After 20 days of culture, the tissue was naturally separated into the cambium, the phloem and the epidermis, as shown in FIG. 3B.

### Example 3: Proliferation of stem cells derived from cambium of family Asteraceae and observation of characteristics of the stem cells

The cambium-derived stem cells isolated in Example 1 above were placed in a flask containing the liquid medium shown in Table 2 below. Then, the stem cells in the flask were cultured in a rotating shaker under dark conditions at 100 rpm at 25 ±1 °C. The interval of subculture was set at 2 weeks, such that the cultured cells could always be maintained in high activity in the exponential growth phase.

**[Table 2]**

| Suspension medium (medium 2) | | |
|---|---|---|
| | Composition | Contents(mg/L) |
| | Ca(NO₃)₂ | 471.26 |
| | NH₄NO₃ | 400 |
| | MgSO₄· 7H₂O | 180.54 |
| | MnSO₄· 4H₂O | 22.3 |
| | ZnSO₄· 7H₂O | 8.6 |
| Inorganic salts | CuSO₄· 5H₂O | 0.25 |
| | CaCl₂· 2H₂O | 72.5 |
| | K₂SO₄ | 990 |
| | Na₂MoO₄· 2H₂O | 0.25 |
| | H₃BO₃ | 6.2 |
| | KH₂PO₄ | 170 |
| | FeNaEDTA | 36.7 |
| | Myo-inositol | 200 |
| | Thiamine-HCl | 20 |
| Vitamin | Nicotinic acid | 2 |
| | Pyridoxine-HCl | 2 |
| | L-ascorbic acid | 50 |
| | Citric acid | 75 |
| | L-aspartic acid | 133 |
| Amino acid | L-arginine | 175 |
| | Glycine | 75 |
| | Proline | 115 |
| Hormone | α-Naphtalene acetic acid | 2 |
| Sucrose | | 30,000 |

The degree of aggregation of the cells was observed with biological microscope CX31 (Olympus, Japan). As a result, as shown in FIG. 4A, it was observed that the stem cells according to the present invention included a large number of single cells during suspension culture, and some of the cells were present as small-sized cell aggregates. However, in a control, a callus derived from the hypocotyl tissue of artemisia were aggregated as shown in FIG. 4B.

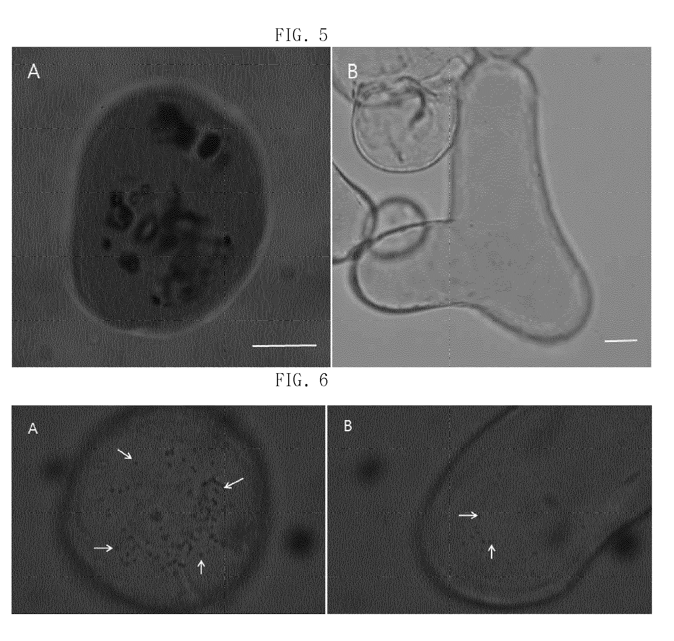

Meanwhile, the vacuoles of the cells were observed after staining with neutral red, and as a result, as shown in FIG. 5A, the stem cells according to the present invention were morphologically characterized by abundant vacuoles immediately after isolation. As pressure applied to stem cells during a culture process decreases, the isolated stem cells change to a morphology having only 1 or 2 vacuoles. Such stem cells that changed to a morphology having a small number of vacuoles show a characteristic in that they will have abundant vacuoles when pressure is applied thereto. This characteristic also appears in undifferentiated cells in plants by factors such as pressure, suggesting that the stem cells according to the present invention are in an undifferentiated state. On the contrary, a callus derived from the hypocotyl tissue of artemisia generally include one or three vacuoles as shown in FIG. 5B and have a maximum of 5 vacuoles in very rare cases.

Meanwhile, the stem cells according to the present invention were observed with optical microscope BX41TF. As a result, a large number of mitochondria having a very high activity in terms of movement could be observed in the stem cell. FIG. 6A shows that the stem cell according to the present invention has a large number of mitochondria. However, as shown in FIG. 6B, this characteristic could not be observed in callus derived from the hypocotyl of artemisia.

Meanwhile, in order to examine the possibility of large scale cell culture, the artemisia cambium-derived stem cells according to the present invention were cultured in an airlift bioreactor (Samsung Science Co., Ltd., Korea) having an internal volume of 3 L. The culture was carried out in the liquid medium of Table 2 under dark conditions at 25±1 °C and an aeration rate of 0.1-0.3 vvm.

**[Table 3]**

| Results of measurement of growth rate and GI | | |
|---|---|---|
| Cell lines | Growth rate (folds) | GI |
| Stem Cell | 2.87 | 1.87 |

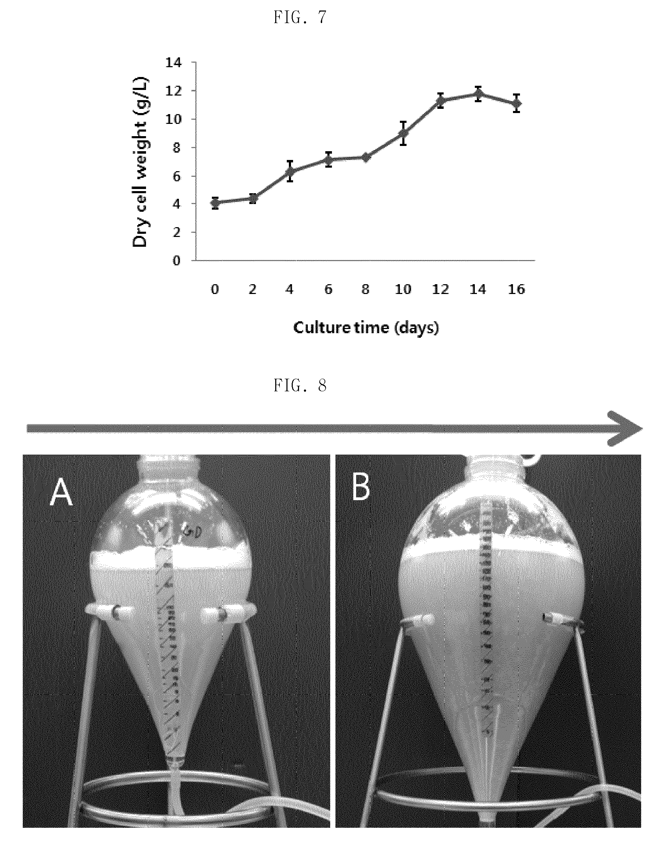

As a result, as shown in FIG. 7, the amount of inoculation of the cambium-derived stem cells according to the present invention was an average of 4.1 g/L at the start of culture, and the maximum dry weight of the cells reached 11.8 g/L at 14 days of culture. Thus, as can be seen in Table 3 above, the growth rate of the cells was increased 2.87-fold at 14 days of culture, and the GI (growth index = (maximum DCW - initial DCW) / initial DCW) of the cells was as high as 1.87. According to the results of studies conducted by N.B. Paniego et al. (N.B. Paniego & A.M. Giulietti, Plant Cell Tissue Organ Cult., 36: 163, 1994), the growth rate of callus, dedifferentiated tissue, was increased about 1.3-fold from about 3 mg (DW) at initial inoculation to about 4 mg (DW) at 15 days of culture, even when the callus was cultured in a flask known to be advantageous for the growth of cells compared to a bioreactor. This suggests that a cell growth rate of 11.8 g/L (2.87 fold) in the 3-L bioreactor is very excellent.

In the case of conventional reactors, cell viability rapidly decreases due to growth ring production in the reactor, plant cell aggregation during culture, and the sensitivity of hard cell walls to shear stress. However, the cambium-derived stem cells formed a very small growth ring area in the bioreactor during culture, and the ring formed on the internal wall thereof was simply eliminated when a simple stimulus was applied to the incubator. Also, it was shown that the cambium-derived stem cells had low aggregation and contained a large number of vacuoles, and thus had low sensitivity to shear stress, such that cell viability did not decrease.

**[Table 4]**

| Large-scale culture process | | |
|---|---|---|
| | Bioreactors | Max. dry cell weight (g/L) |
| A | 3L air-lift bioreactor | 10.1 |
| B | 20L air-lift bioreactor | 11.8 |

In addition, as can be seen in Table 4 and FIG. 8, when the stem cells were cultured in each of the 3-L air-lift bioreactor and the 20-L air-lift bioreactor for 12 days, it was shown that the amount of dry cells per liter in the 20-L bioreactor rather increased.

In other words, it was seen that the cambium-derived stem cells according to the present invention had low sensitivity to shear stress in the bioreactor for scale-up culture, and thus could be produced rapidly in large amounts in the bioreactor. Accordingly, it could be seen that the cambium-derived stem cells according to the present invention had lower sensitivity to shear stress than cell derived from the dedifferentiated callus of Artemisia.

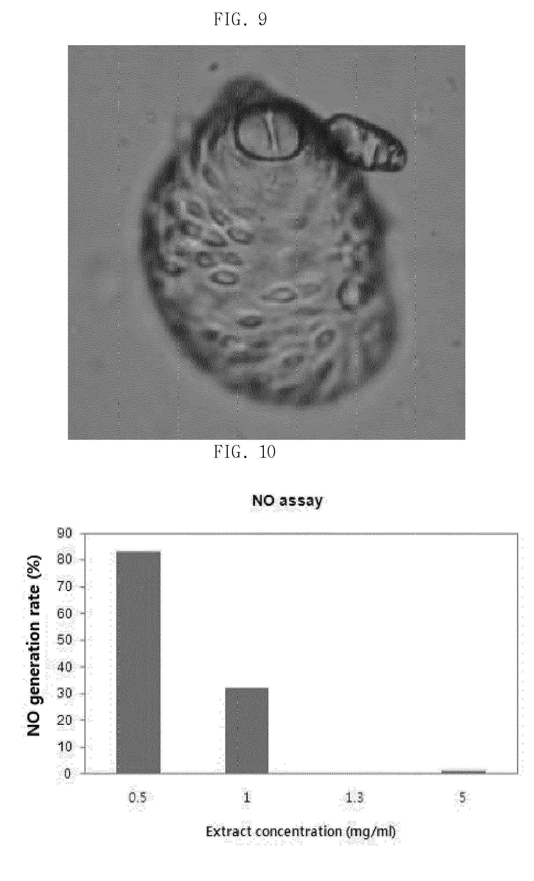

In addition, the characteristics of plant stem cells include self-renewal ability and pluripotency. Thus, in order to induce the Artemisia cambium-derived stem cells to differentiate into tracheary elements, the stem cells were cultured in each of MS, B5 and WPM media containing picloram at concentrations of 0.1, 0.5 and 1 mg/L, respectively, at 25±1 °C under dark conditions. As a result, as can be seen in FIG. 9, the cambium-derived stem cells differentiated into tracheary elements.

### Example 4: Treatment with elicitor and preparation of extract of stem cells derived from cambium of plant of family Asteraceae

### 4-1: Treatment with elicitor

The stem cells, which have been suspension-cultured for 14 days as described in Example 3, were divided into the following two groups for experiments: (1) the cell line that was suspension-cultured for 14 days (growth Phase); and (2) the cell line which was suspension-cultured for 14-days and then cultured in a medium containing 3-5 wt% (g/L) raw sugar and 100 µM methyl jasmonate in sterile water at a dark condition for 14 days (elicitation phase).

### 4-2: Preparation of extract

Each of the two cell lines from which the culture medium has been removed was freeze-dried. Then, 2 g of each freeze-dried cell line (dry) was stirred in 50 ml of 80% ethanol with stirring at 15 °C for 48 hours for dissolution purpose. After completion of the dissolution, each of the cell solutions was centrifuged at 3,000 rpm for 20 minutes, and each of the supernatants was freeze-dried. Each of the resulting extract powders was dissolved in PBS, thereby obtaining an ethanol extract of each of the two cell lines.

### Example 5: Examination of inhibitory effect on production of NO (nitric oxide) - examination of anti-inflammatory effect (1)

NO analysis is a method of measuring macrophage activity and is a method of indirectly measuring activity associated with phagocytosis that is one of the important functions of macrophages. The inhibitory effect of each ethanol extract of Example 4-2 on NO production caused by LPS (lipopolysaccharide) treatment on RAW264.7 cells was measured by the amount of NO dissolved in each of the supernatants.

First, a 24-well culture plate was pretreated with RAW264.7 cells (2 x 10⁵ cells/ml) and varying concentrations of each sample and incubated for 30 minutes. Then, the culture plate was treated with 1 µg/ml of LPS and incubated for 24 hours. Then, 100 µℓ of the cell culture medium was allowed to react with 100 µℓ of Griess reagent at room temperature for 15 minutes under dark conditions, after which the absorbance at 540 nm was measured using a microplate reader (Packard, USA). The concentrations of NO in the wells containing the cell culture medium and LPS were expressed as percentages relative to that in a control containing only the culture medium.

**[Table 5]**

| | 0.5mg/ml | 1mg/ml | 1.3mg/ml | 5mg/ml |
|---|---|---|---|---|
| Growth Phase | 100 | 97 | 90 | 73 |
| Elicitation phase | 83 | 32 | 0 | 1.4 |

As a result, as can be seen in Table 5 above, the ethanol extract (growth phase) of the artemisia cambium-derived stem cells inhibited the NO production inducted by LPS in a concentration-dependent manner. In particular, the ethanol extract (elicitation phase) obtained from the cell line treated with raw sugar and methyl jasmonate as elicitors showed an inhibitory rate of 100% at a concentration of 1.3 mg/ml. Such test results suggest that the cambium-derived stem cells according to the present invention have excellent anti-inflammatory effects. FIG. 10 graphically shows the degree of inhibition of NO production by the ethanol extract treated with the elicitors.

### Example 5: Examination of inhibitory effect on expression of COX-2 - examination of anti-inflammatory effect (2)

In order to examine the effect of inhibiting the expression of COX-2 gene (proinflammatory cytokine), a 6-well culture plate was pretreated with RAW264.7 cells (2 x 10⁶ cells/ml) and varying concentrations of each ethanol extract of Example 4-2 (A: growth phase 0.5 g/ml, B: elicitation phase 0.5 g/ml, C: growth phase 2 mg/ml, D: elicitation phase 2 mg/ml) and incubated for 30 minutes. Then, each of the cell plates was treated with LPS (1 µg/ml) and incubated for 24 hours. Total RNA was isolated from each of the cultured cell lines using TRIzol reagent and was synthesized into cDNA which was then amplified by PCR using the following COX-2 primers.

### <COX-2 primers>

mouse COX2 (forward): 5'-AAGAAGAAAGTTCATTCCTGATCCC-3' (SEQ ID NO: 1)
mouse COX2 (revere): 5'-TGACTGTGGGAGGATACATCTCTCC-3' (SEQ ID NO: 2)

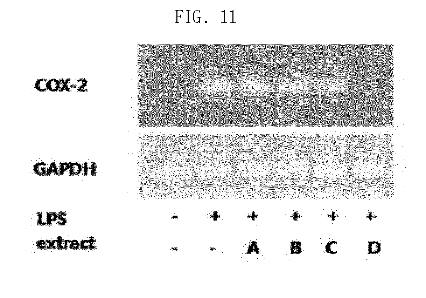

As a result, as can be seen in FIG. 11, the ethanol extract of the cell line treated with the elicitors completely inhibited the expression of COX-2 inflammatory gene, induced by LPS, at a concentration of 2 mg/ml.

### Preparation Example 1: Preparation of pharmaceutical formulations

### Formulation 1-1: Preparation of tablet

100 mg of the stem cell extract prepared in Example 4 was mixed with 100 mg of maize starch, 100 mg of lactose and 2 mg of magnesium stearate, and the mixture was compressed into a tablet according to a conventional tableting method.

### Formulation 1-2: Preparation of capsule formulation

500 mg of the stem cell extract prepared in Example 4 was filled in a soft gelatin capsule to prepare a capsule formulation.

### Formulation 1-3: Preparation of syrup formulation

1 g of the stem cell prepared in Example 1 was mixed with 10 g of isomerized sugar, 5 g of mannitol and a suitable amount of purified water, and the mixture was prepared into 100 ml of a syrup formulation according to a conventional method.

### Formulation 1-4: Preparation of injection solution

200 mg of the stem cell extract prepared in Example 4 was heated and dissolved in 200 mg of physiological saline containing polyoxyethylene hydrogenated castor oil, thus preparing an injection solution containing the stem cell extract at a concentration of 0.1%.

### Preparation Example 2: Preparation of functional foods

### Preparation Example 2-1: Preparation of functional beverage

200 mg of the stem cell prepared in Example 1 was dissolved in 96 ml of water, and then 500 mg of vitamin C as a supplement, 1 g of each of citric acid and oligosaccharide as flavor enhancers and 0.05 g of sodium benzoate as a preservative were added thereto. Then, purified water was added thereto, thus preparing 100 ml of a functional beverage.

### Preparation Example 2-2: Preparation of functional beverage

200 mg of the stem cell extract prepared in Example 4 was dissolved in 96 ml of water, and then 500 mg of vitamin C as a supplement, 1 g of each of citric acid and oligosaccharide as flavor enhancers and 0.05 g of sodium benzoate as a preservative were added thereto. Then, purified water was added thereto, thus preparing 100 ml of a functional beverage.

### INDUSTRIAL APPLICABILITY

As described above, the inventive stem cell line derived from the cambium of a plant of the family *Asteraceae* is isolated in an undifferentiated state without undergoing a dedifferentiation process, such that it can be stably maintained without variations in the growth rate and growth pattern thereof even during long-term culture, and thus can be cultured in large amounts. In addition, the disclosed stem cell line and a culture medium thereof have not only the effect of inhibiting the effect of inhibiting NO production caused by treatment with LPS (lipopolysaccharide), but also the effect of inhibiting the expression COX-2 gene (proinflammatory cytokine), and are thus useful as an effective anti-inflammatory composition.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A stem cell derived from a cambium of family *Asteraceae,* which is an innately undifferentiated cell without going through dedifferentiation.

2. The stem cell derived from a cambium of family *Asteraceae* according to claim 1, wherein the stem cell additionally comprises at least one of the following characteristics:
(a) including a greater number of single cells or including smaller-sized cell aggregates in suspension culture than a dedifferentiated callus of the family *Asteraceae;*
(b) having a large number of vacuoles morphologically;
(c) having a greater number of mitochondria than the dedifferentiated callus of the family *Asteraceae;*
(d) being capable of growing faster and longer than the dedifferentiated callus of the family *Asteraceae;*
(e) having lower sensitivity to shear stress in a bioreactor than the dedifferentiated callus of the family *Asteraceae;* and
(f) having the capability to differentiate into tracheary elements.

3. The stem cell derived from a cambium of family *Asteraceae* according to claim 2, wherein the stem cell comprises two or more characteristics selected from the group consisting of the characteristics (a) to (f).

4. The stem cell derived from a cambium of family *Asteraceae* according to claim 2, wherein the stem cell comprises three or more characteristics selected from the group consisting of the characteristics (a) to (f).

5. The stem cell derived from a cambium of family *Asteraceae* according to claim 2, wherein the stem cell comprises four or more characteristics selected from the group consisting of the characteristics (a) to (f).

6. The stem cell derived from a cambium of family *Asteraceae* according to claim 2, wherein the stem cell comprises five or more characteristics selected from the group consisting of the characteristics (a) to (f).

7. The stem cell derived from a cambium of family *Asteraceae* according to claim 2, wherein the stem cell comprises the characteristics (a) to (f).

8. The stem cell according to claim 1, wherein stem cell is obtained by an isolation method comprising the steps of:
(a) obtaining a tissue comprising a cambium from a plant of the family *Asteraceae;*
(b) culturing the obtained cambium-comprising tissue in a medium; and
(c) isolating a cell from the cambium, thereby obtaining a stem cell derived from the cambium.

9. The stem cell according to any one claim among claims 1 to 8, wherein the plant of the family *Asteraceae* is genus *Artemisia* or genus *Chrysanthemum.*

10. The stem cell according to any one claim among claims 1 to 8, wherein the plant of the family *Asteraceae* is Artemisia or chrysanthemum.

11. A method of isolating a stem cell derived from a cambium of family *Asteraceae,* the method comprising the steps of:
(a) obtaining a tissue comprising a cambium from a plant of the family *Asteraceae;*
(b) culturing the obtained cambium-comprising tissue in a medium; and
(c) isolating a cell from the cambium, thereby obtaining a stem cell derived from the cambium.

12. The method according to claim 11, wherein the medium of the step (b) comprises auxin.

13. The method according to claim 12, wherein the medium comprises 1∼5mg/L of auxin.

14. The method according to claim 11, wherein the step (c) comprises isolating cells from the cambium after separating the cultured cambium from an amorphous callus layer derived from tissues other than the cambium, thereby obtaining the stem cell derived from the cambium.

15. The method according to claim 11, wherein the plant of the family *Asteraceae* is genus *Artemisia* or genus *Chrysanthemum.*

16. The method according to claim 11, wherein the plant of the family *Asteraceae* is Artemisia or chrysanthemum.

17. An anti-inflammatory composition containing any one or more of the stem cell derived from the cambium of the family *Asteraceae* of any one claim among claims 1 to 8; an extract thereof; a lysate thereof; and a culture thereof.

18. The anti-inflammatory composition according to claim 17, wherein the culture is obtained by additionally culturing the stem cell in a medium containing 3-5 wt% of raw sugar or sugar; or one or more substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

19. The anti-inflammatory composition according to claim 17, wherein the plant of the family *Asteraceae* is genus *Artemisia* or genus *Chrysanthemum.*

20. The anti-inflammatory composition according to claim 17, wherein the plant of the family *Asteraceae* is artemisia or chrysanthemum.

21. A functional food for preventing or alleviating inflammation, which contains any one or more of the stem cell derived from the cambium of the family *Asteraceae* of any one claim among claims 1 to 8; an extract thereof; a lysate thereof; and a culture thereof.

22. The functional food for preventing or alleviating inflammation according to claim 21, wherein the culture is obtained by additionally culturing the stem cell in a medium containing 3-5 wt% of raw sugar or sugar; or one or more substance selected from the group consisting of methyl jasmonate, fungal extract, bacterial extract, yeast extract, chitosan, glucomannan, glucan, phenylalanine, benzoic acid, salicylic acid, arachidonic acid, STS, mevalonalonate N-benzolyglycine, ABA, SNP, IPP, BHT, CCC, ethephon, hippuric acid, ammonium ceric nitrate, AgNO₃, vanadyl sulfate, p-aminobenzoic acid, brassinosteroids, sodium alginate, and sodium acetate.

23. The functional food for preventing or alleviating inflammation according to claim 21, wherein the plant of the family *Asteraceae* is genus *Artemisia* or genus *Chrysanthemum.*

24. The functional food for preventing or alleviating inflammation according to claim 21, wherein the plant of the family *Asteraceae* is artemisia or chrysanthemum.
